# EUROPÄISCHE PATENTSCHRIFT

(11) **EP 1 501 408 B1**
(45) Veröffentlichungstag und Bekanntmachung des Hinweises auf die Patenterteilung: **21.10.2009**
(21) Anmeldenummer: 03727441.2
(22) Anmeldetag: 06.05.2003
(51) Int. Cl.: A61B 5/00, G06F 19/00

(54) **VORRICHTUNG ZUR MESSUNG, AUFZEICHNUNG UND SPEICHERUNG VON PHYSIOLOGISCHEN DATEN UND BEFINDLICHKEITSWERTEN EINES PATIENTEN**
DEVICE FOR THE MEASUREMENT, RECORDING AND STORAGE OF PHYSIOLOGICAL DATA AND THE SENSITIVITY VALUES OF A PATIENT
DISPOSITIF POUR MESURER, ENREGISTRER ET STOCKER DES DONNEES PHYSIOLOGIQUES ET DES VALEURS DE SENSIBILITE D'UN PATIENT

(30) Priorität: 08.05.2002 DE 10221324
(43) Veröffentlichungstag der Anmeldung: 02.02.2005
(73) Patentinhaber: Von Zitzewitz, Falk, 71638 Ludwigsburg (DE)
(72) Erfinder: Von Zitzewitz, Falk, 71638 Ludwigsburg (DE)
(74) Vertreter: Heumann, Christian
(86) Internationale Anmeldenummer: PCT/EP2003/004725
(87) Internationale Veröffentlichungsnummer: WO 2003/094709

(56) Entgegenhaltungen:
- WO-A-01/78577
- WO-A-01/93753
- WO-A-97/37585
- US-A- 5 513 646

## Beschreibung

Die Erfindung bezieht sich auf eine Vorrichtung zur Messung, Aufzeichnung und Speicherung von physiologischen, mittels Sensoren am Körper eines Patienten gemessenen Daten und von vom Patienten eingegebenen Befindlichkeitswerten. Eine solche Vorrichtung wurde durch die WO 96/25877 bekannt. Die Erfindung betrifft auch ein Verfahren zur Überwachung des Gesundheitszustandes eines Patienten, insbesondere im Hinblick auf seine physiologischen, mittels Sensoren messbaren Werte und seine subjektiven Befindlichkeitswerte.

In der WO 96/25877 ist ein tragbares Gesundheitsüberwachungsgerät beschrieben, welches einerseits eine Vielzahl von physiologischen Daten eines Patienten mittels geeigneter Sensoren misst und speichert sowie andererseits auch subjektive Daten des Patienten, z B. Schmerz, Stimmung oder Angst abfragt. Zu den objektiven, d. h. physiologischen Daten gehören z. B. die Herzfrequenz, messbar mittels EKG-Elektroden, oder die Atmung, messbar mittels eines Dehnungsmesssensors an einem elastischen Brustgurt. Die subjektiven und objektiven Werte werden periodisch und parallel zueinander, d. h. teilweise synchron gemessen, als individuelle Gesundheitsgeschichte des Patienten aufgezeichnet und dann vom Arzt verwertet. Nachteilig bei diesem Gerät ist es - aus ärztlicher Sicht - dass durch diese Gesundheitsgeschichte eine eventuelle Abhängigkeit zwischen den objektiven und den subjektiven Daten nicht eindeutig erkennbar wird.

Durch die EP-B 892 619 des Anmelders wurde ein tragbares Gerät zur Registrierung, Anzeige und Speicherung von subjektiven Befindlichkeitswerten bekannt. Dabei gibt der Patient jeweils dann, wenn er eine Änderung seiner Befindlichkeit, wahrnimmt, seinen akuten, subjektiv empfundenen Befindlichkeitswert über eine erste oder zweite Taste ein, d. h. er registriert die Änderung seines Zustandes ins Positive oder Negative. Außerdem gibt er vorher über eine dritte Taste den Zeitpunkt, der Medikamenteneinnahme ein, so dass als Ergebnis eine Funktion der Befindlichkeit des Patienten in Abhängigkeit von der Medikamenteneinnahme vorliegt. Ferner sind bei diesem bekannten Gerät diverse Messfühler für physikalisch messbare Patientendaten vorgesehen, also z. B. zur Messung des Blutsauerstoff bzw. -zuckergehaltes oder der Herzfrequenz. Diese Werte werden automatisch, d. h. ohne Zutun des Patienten gemessen und registriert, d. h. auch unabhängig von der Eingabe der Befindlichkeitswerte durch den Patienten. Auch hier ist eine mögliche Abhängigkeit der subjektiven und objektiven Daten voneinander nicht ohne weiteres aus der Aufzeichnung erkennbar.

Die WO 01/78577 A2 offenbart eine Vorrichtung zur Messung von physiologischen Daten mittels Sensoren sowie eine Alarmeinrichtung, welche bei Überschreiten von bestimmten Soll-Werten eine Alarmsituation erkennt und einen Alarm auslöst. Darüber hinaus erlaubt die bekannte Vorrichtung in Form einer Mikroprozessoreinheit, dass ein Patient nicht messbare Daten, z. B. seine Stimmung (mood) eingibt, beispielsweise über eine Tastatur. Wann diese Stimmungswerte vom Patienten eingegeben werden sollen, bleibt dem Patienten überlassen.

Die WO 01/93753 A1 betrifft eine Vorrichtung zur Messung und Aufzeichnung von physiologischen Daten eines Patienten, verbunden mit einer Überwachungseinrichtung, die den Patienten zu bestimmten Handlungen, z. B. zur Einnahme von Medikamenten, zum Essen oder auch zur Durchführung einer zusätzlichen Messung auffordert.

Durch die US-A 5 513 646 wurde ein tragbares Gerät zur Überwachjung der Atmung einer Person bekannt, wobei bei kritischen Atemzuständen, z. B. einer Apnoe ein Alarm ausgelöst und an eine Überwachungsstelle gesendet wird. Die Person wird über den Alarm informiert und kann einen Alarm auch selbst absetzen.

Aufgabe der vorliegenden Erfindung ist es, ein Gerät der eingangs genannten Art dahingehend zu verbessern, dass eine eventuelle

Abhängigkeit der subjektiven von den objektiven Daten aus der Aufzeichnung der Daten für den Arzt erkennbar wird. Darüber hinaus ist es Aufgabe der Erfindung, ein Verfahren zur Überwachung des Gesundheitszustandes eines Patienten hinsichtlich seiner objektiven und subjektiven Werte anzugeben.

Diese Aufgabe wird durch die Merkmale der unabhängigen Patenansprüche 1 und 14 gelöst. Danach werden einerseits die physiologischen Daten des Patienten kontinuierlich oder in Zeitintervallen gemessen und mit vorgegebenen Sollwerten für den Patienten verglichen. Bei Überschreiten dieser Sollwerte wird ein Alarm ausgelöst, der den Patienten auffordert, seine Befindlichkeitswerte unmittelbar in das Gerät einzugeben und ein bestimmtes Medikament einzunehmen. Die Alarmvorrichtung kann ein akustisches, visuelles, taktiles Signal abgeben, und die entsprechende Aufforderung oder Anweisung an den Patienten kann auf einem Display des Gerätes vom Patienten abgelesen, als Sprachausgabe gehört oder als taktile Botschaft gefühlt werden. Aufgrund dieses Alarms gibt der Patient also bei jeder Extremsituation (Sollwertüberschreitung) und im nachfolgenden Zeitraum seine Befindlichkeitswerte ein, d. h. der Arzt kann später aus der Aufzeichnung erkennen, in welchem Zustand sich der Patient befunden hat - z. B. wenn das EEG einen so genannten Spike registriert hat.

Nach einer vorteilhaften Ausgestaltung der Erfindung weist die Alarmvorrichtung eine elektronische Vergleichseinheit auf, in welcher die individuellen Sollwerte des Patienten gespeichert und mit den gemessenen Werten verglichen werden. Vorteilhafterweise können die gemessenen Daten per Funk an eine entsprechende Empfängerstation der Alarmvorrichtung übermittelt werden. Dies erspart dem Patienten eine umständliche "Verdrahtung".

Nach einer vorteilhaften Weiterbildung der Erfindung können die beim Patienten ermittelten Messwerte und die von ihm eingegebenen Befindlichkeitswerte drahtlos an eine Zentrale übermittelt werden. Dadurch wird erreicht, dass Veränderungen des Gesundheits- und Befindlichkeitszustandes des Patienten auch vom behandelnden Arzt (oder einer vergleichbar ausgebildeten Person) ohne Verzögerung verfolgt werden können; der Arzt kann gegebenenfalls sofort reagieren und notwendige Maßnahmen, z. B. Änderung der Medikation oder Einweisung in eine Klinik veranlassen.

Nach einer vorteilhaften Weiterbildung der Erfindung werden die gemessenen Werte und die eingegebenen Befindlichkeitswerte zunächst an eine stationäre oder mobile Zwischenstation und von dort drahtlos oder per Datenleitung an die Zentrale übermittelt. Die Zwischenstation dient als Verstärker und befindet sich in unmittelbarer Nähe oder im näheren Umgebungsbereich des Patienten, z. B. stationär in seiner häuslichen Umgebung. Durch die Zwischenstation wird der Vorteil erreicht, dass auch größere Entfernungen zwischen dem Patienten und der Zentrale wirksam überbrückt werden können, ohne große Sendeleistungen am Patienten zu installieren, die der Patient als Gewicht mit sich herumtragen muss. Die Zwischenstation kann auch mobil in einem Fahrzeug als geeignete Empfangs- und Sendestation mitgeführt oder installiert sein, wobei die Weiterleitung der Daten von der Zwischenstation an die Zentrale auch per Datenleitung, sofern vorhanden, erfolgen kann.

Nach einer weiteren vorteilhaften Ausgestaltung der Erfindung können die Sollwertgrenzen der Messfühler (Sensoren) und die der Befindlichkeitseingaben sowie die ärztlichen Vorgaben für die Medikamenteneinnahme durch den Patienten von der Zentrale aus - gegebenenfalls über die Zwischenstation - verändert werden. Durch diese zentrale Überwachung und Steuerung, z. B. durch den behandelnden Arzt kann in geeigneter Weise und unverzüglich auf kritische Änderungen des Zustandes beim Patienten reagiert werden.

Nach einer weiteren vorteilhaften Ausgestaltung der Erfindung können die vom Patienten einzugebenden Befindüchkeitswerte über eine Multifunktionstaste, einen so genannten Joystick, oder über ein so genanntes Berührfeld ("touch screen") eingegebenen werden. Die an sich bekannte Multifunktionstaste erlaubt eine einfache Bedienung mit einem Finger (dem Daumen oder Zeigefinger). Die Eingabe über ein Berührfeld ist von tragbaren Computern (handelsüblich unter der Bezeichnung "Palm" oder hand-held-computer) bekannt und erfolgt vorzugsweise über einen speziellen Stift für den "touch screen" oder direkt mit den Fingern. Diese erweiterte Eingabemöglichkeit hat den Vorteil, dass auch weitere für den Patienten relevante Daten eingegeben werden können, die bereits als Optionen hinterlegt und aufrufbar sind.

Ausführungsbeispiele der Erfindung sind in der Zeichnung dargestellt und werden im Folgenden näher beschrieben. Es zeigen
- Fig. 1: einen Patienten mit Sensoren und Alarmvorrichtung sowie Zwischenstation und Zentrale,
- Fig. 2: ein Flussdiagramm für die Steuerfunktion einer Zentrale,
- Fig. 3: ein Gerät zur Eingabe von Befindlichkeitswerten über eine Multifunktionstaste und
- Fig. 4: ein weiteres Gerät zur Eingabe von patientenrelevanten Daten über ein Berührfeld.

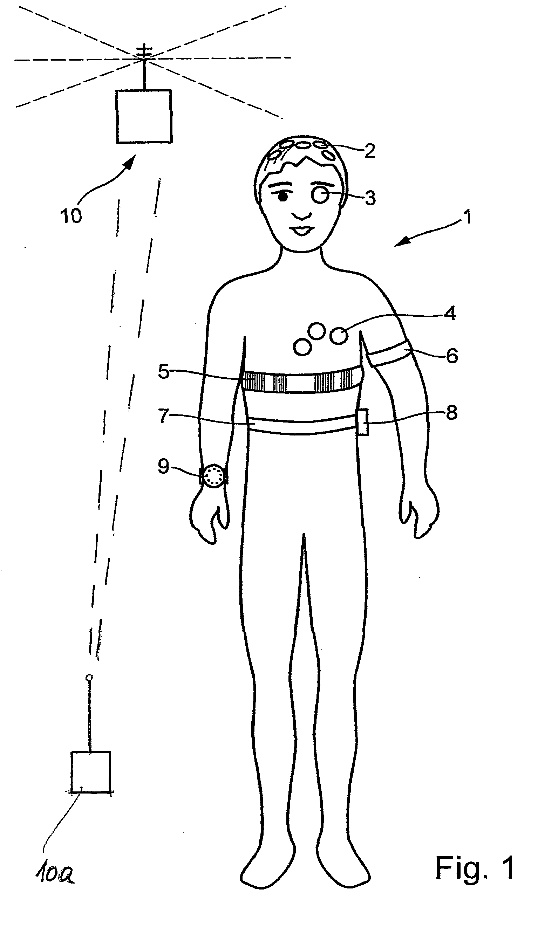

Die Fig. 1 zeigt einen Patienten 1, der eine Vielzahl von Sensoren zur Messung von physiologischen Patientendaten trägt. Im Einzelnen handelt es sich um folgende Sensoren: auf dem Kopf des Patienten sind EEG-Elektroden 2 zur Messung der Gehimströme angebracht. An den Augen des Patienten ist ein so genanntes ODG 3 zur Messung des Augendruckes angeordnet. Im Herzbereich sind EKG-Elektroden 4 auf der Haut des Patienten befestigt. Unterhalb der Elektroden 4 trägt der Patient einen elastischen Brustgurt 5, an welchen nicht dargestellte Dehnungssensoren zur Messung der Atmung angebracht sind. Am Oberarm trägt der Patient eine Manschette 6 zur Messung des Blutdruckes. Weitere nicht dargestellte Sensoren sind möglich, um weitere physiologische Daten des Patienten, z. B. den Blutzuckergehalt zu erfassen.

Im Hüftbereich trägt der Patient mittels eines Gürtels 7 eine so genannte Box 8, die eine nicht näher dargestellte Alarmvorrichtung sowie eine Empfangsstation zur Aufnahme der von den Sensoren abgegebenen Signale enthält. Schließlich trägt der Patient an seinem Handgelenk ein Befindlichkeitsmessgerät 9, wie es aus dem eingangs genannten Stand der Technik (EP-B 892 619) bekannt ist.

Die Wirkungsweise der gesamten Vorrichtung ist Folgende: Die o. e. physiologischen Werte des Patienten werden kontinuierlich oder in Zeitintervallen durch die Sensoren 2, 3, 4, 5, 6 gemessen und per Funk an die Box 8 übermittelt (die Übermittlung kann auch auf anderem Wege, z. B. per Kabel oder andere Datenübertragungsmittel erfolgen). Dort werden sie empfangen, registriert, gespeichert und mit den zugehörigen, für den Patienten festgelegten Sollwerten verglichen. Übersteigt ein gemessener Wert, z. B. die Herzfrequenz den vorgegebenen Sollwert für den Patienten, so wird über die Box 8 ein für den Patienten akustisch, visuell oder taktil wahrnehmbarer Alarm ausgelöst. Der Patient wird jetzt über ein nicht dargestelltes Display (lesbare Flüssigkristallanzeige) aufgefordert, seine akuten Befindlichkeitswerte, z. B. nach einer vorgegebenen Skala in das Gerät 9 einzugeben. Zusätzlich kann der Patient, ebenfalls durch eine Anweisung per Display, zur Einnahme eines Medikaments oder zur Durchführung einer speziellen Therapie aufgefordert werden. Die Anweisung kann statt über ein Display auch sprachlich über ein nicht dargestelltes Sprachmodul oder taktil über ein haptisches Modul erfolgen. Durch die Alarmvorrichtung in der Box 8 kann somit sichergestellt werden, dass die Befindlichkeitswerte, d. h. die subjektiv vom Patienten wahrgenommenen Werte gleichzeitig mit der Überschreitung der Sollwerte registriert und gespeichert werden. Dies kann der Arzt aus der Aufzeichnung der Box 8 entnehmen.

In der Zeichnung ist auch eine zentrale Empfangs- und Kontrollstation 10 dargestellt, an welche sowohl die am Patienten gemessenen physiologischen Messwerte als auch die eingegebenen Befindlichkeitswerte drahtlos übermittelt werden. Diese Zentralstation 10 (im Folgenden Zentrale genannt) ist dann auch in der Lage, die Sollwertgrenzen - für den Patienten zu korrigieren, d. h. nach oben oder nach unten zu verschieben. Dies kann bei bestimmten Zustandsänderungen des Patienten erforderlich werden. Darüber hinaus kann diese Zentralstation auch die Vorgaben für den Patienten zur Medikamenteneinnahme oder zur Einleitung einer speziellen Therapie durch drahtlose Übermittlung ändern. Diese zentrale Empfangs- und Überwachungsstation kann somit in kritischen Zuständen des Patienten sofort, also z. B. in Notfallsituationen mit gezielten Maßnahmen reagieren.

Alternativ kann die Datenübertragung an die Zentrale 10 auch über eine in der Nähe des Patienten installierte Zwischenstation 10a erfolgen, wodurch eine Verstärkung der Sendeleistung und eine Erhöhung der Reichweite erreicht wird. Die Zwischenstation 10a kann sich z. B. im häuslichen Umgebungsbereich des Patienten befinden, aber auch im Auto, im Zug, im Flugzeug oder auf dem Schiff, d. h. stationär oder mobil. Die Datenübertragung von der Zwischenstation 10a an die Zentrale 10 erfolgt drahtlos oder per Datenleitung, z. B. über das Internet. Ebenso erfolgt die Datenrückübertragung von der Zentrale 10 auf die Zwischenstation 10a und von dort auf die Box 8.

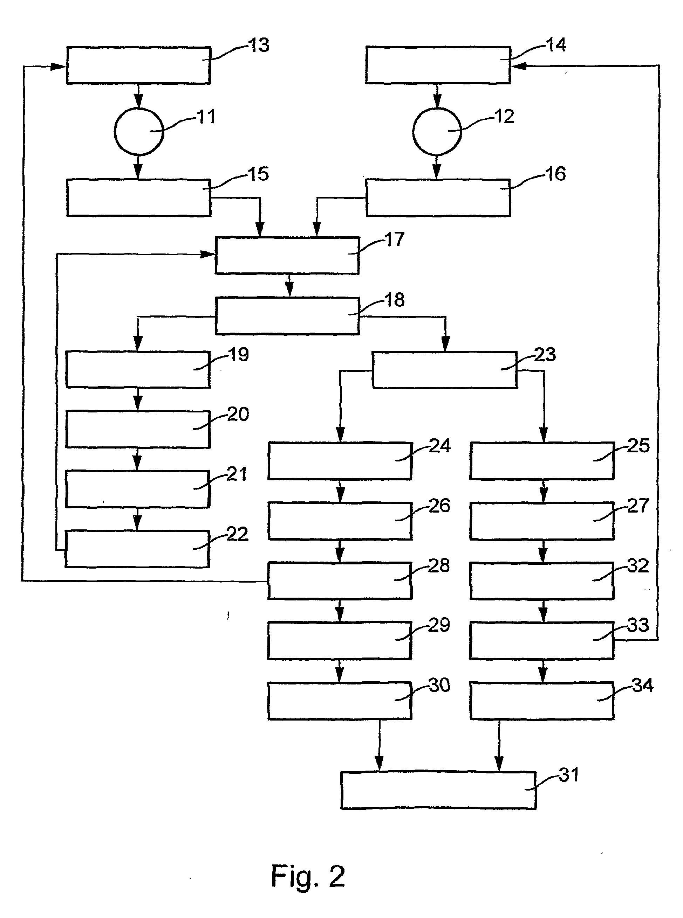

Die Funktion der Zentrale 10 ist in einem Flussdiagramm in Fig. 2 dargestellt, aus dem die Entscheidungsabläufe und Aktionen deutlich werden, dargestellt durch einzelne mit Bezugszahlen versehene Felder. Diese Bezugszahlen sind nachfolgend in Klammern gesetzt.

**Fig. 2** zeigt zwei Alarmvorrichtungen (11, 12), die bei einer Sollwertüberschreitung der durch die Sensoren gemessenen Werte (13) bzw. bei Überschreitung der Sollwerte der vom Patienten eingegebenen Befindlichkeitswerte (14) ansprechen. Die überschrittenen Werte (15, 16) werden jeweils in der Zentrale registriert, wo eine Aufforderung zur ärztlichen Beurteilung (17) erfolgt. Der Arzt trifft eine Entscheidung (18), z. B. *gibt er* eine Therapieanweisung (19), die beim Patienten im Display (20) angezeigt wird. Der Patient bestätigt dann die Therapieanweisung (21), was anschließend an die Zentrale (22) zurückgemeldet wird. Alternativ oder kumulativ zu der obigen Entscheidung (19) kann der Arzt (oder eine entsprechend ausgebildete Person) einen neuen Sollwert vorgeben (23), der je nach dem, ob es sich um einen physiologischen oder Befindlichkeitswert handelt, an die Sensoren (24) oder an die Befindlichkeitsregistrierung (25) übermittelt wird. Es erfolgt dann eine Neueinstellung der Sensoren mit neuem Sollwert (26) bzw. eine Neueinstellung (27) am Patienten. Mit den neuen Sollwerten erfolgt dann eine neue Messung (28) durch die Sensoren, und diese Messwerte am Patienten werden registriert (29). Die Messergebnisse werden an die Zentrale übertragen (30) und im Archiv gespeichert (31). Parallel zur Übermittlung der geänderten Sollwerte (26) an die Sensoren läuft die Übermittlung der neuen Befindlichkeitssollwerte: nach der Neueinstellung am Patienten (27) wird dieser zur Neubewertung aufgefordert (32) und die neuen Befindlichkeitswerte werden registriert (33). Auch diese Befindlichkeitswerte werden an die Zentrale übertragen (34), wo sie ebenfalls gespeichert werden (31).

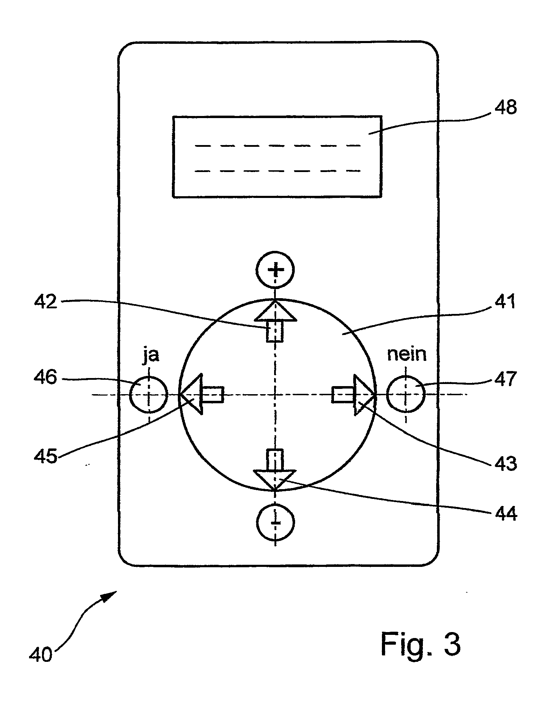

Fig. 3 zeigt ein vereinfachtes Gerät 40 zur Eingabe von Befindlichkeitswerten - es stellt eine neue Ausführungsform des oben erwähnten Gerätes 9 dar, welches durch den Stand der Technik des Anmelders bekannt ist. Bei diesem bekannten Gerät nach der EP-B 0 892 619 sind mindestens drei Tasten vorgesehen, eine so genannte M-Taste zur Registrierung des Zeitpunktes der Medikamenteneinnahme, eine zweite so genannte P-Taste zur Registrierung einer positiven Wirkung und eine dritte so genannte N-Taste zur Registrierung einer negativen Wirkung. Möglicherweise ist noch eine vierte, so genannte W-Taste für zusätzlich auftretende Ereignisse und deren Bewertung vorgesehen. Bei dem neuen Gerät 40 sind alle vier Tasten in einer Multifunktionstaste, d. h. in einem in "vier Himmelsrichtungen" schwenkbaren Knopf 41, einem so genannten Joystick zusammengefasst. Rechts und links neben dem Joystick 41 sind zwei zusätzliche Tasten 46, 47 angeordnet, und zwar für die Bestätigung (Taste 46) oder für die Löschung (Taste 47) einer Eingabe. Die Taste 46 kann daher mit "ja" und die Taste 47 mit "nein" bezeichnet werden. Der vorgenannte Joystick 41 ist an sich aus mobilen Navigationsgeräten (GPS) bekannt; er weist vier Pfeile 42, 43, 44, 45 auf, die jeweils in eine Himmelsrichtung weisen. Über dem "Nord-Pfeil" ist ein Plus eingezeichnet und über dem "Süd-Pfeit" 44 ein Minus. Der Patient kann somit durch Drücken mit seinem Daumen (oder beispielsweise mit dem Zeigefinger) auf den "Nord-Pfeil" 42 eine positive Wirkung registrieren, die auf einem Display 48 sichtbar wird. Analog kann er durch Drücken des "Süd-Pfeils" 44 eine negative Wirkung registrieren. Die beiden anderen Pfeile 43 und 45 können analog zu den bekannten M- und W-Tasten benutzt werden:

Beispielsweise führt der Druck auf den Pfeil 43 des Joysticks zur "W-Funktion", also zur Wahl einer Befindlichkeit 1, ein weiterer Druck auf den Pfeil 43 zur Befindlichkeit II, ein weiterer Druck auf den Pfeil 43 zur Befindlichkeit III. Ein Druck in anderer Richtung, z. B. auf den Pfeil 42 führt zur Auswahl einer positiven Stufe I, ein weiterer Druck auf den Pfeil 42 zu einer positiven Stufe II und so weiter; dagegen führt ein Druck auf den gegenüberliegenden Pfeil 44 zunächst zu einer positiven Stufe I, d. h. zur Subtraktion von dem eingegebenen Wert. Durch weiteres Drücken des Pfeils für 44 - gelangt man zu den negativen Stufen I, II und so weiter. Bei Einstellung der Befindlichkeit III (beispielsweise Kopfschmerzen) und negativer Stufe II (beispielsweise stark) wird bei nachfolgend gedrückter Taste 46 "Kopfschmerzen: stark" registriert; mit der Taste 47 ist diese Registrierung wieder löschbar, so dass eine andere Stufe oder auch eine andere Befindlichkeit wählbar ist. Der Druck auf den Pfeil 45 des Joystick führt über Befindlichkeit II und III zur Medikamentenwahl, ähnlich wie bei der Befindlichkeit, wobei ein Druck auf den Pfeil 45 zu Medikament I, dann bei erneutem Druck auf den Pfeil 45 zu Medikament II und so weiter führen. Die Bestätigungstaste 46 registriert die Einnahme des Medikaments, die Löschtaste 47 hebt diese Registrierung wieder auf.

Bei Einstellung des Medikaments II führt ein Druck auf den Pfeil 42 zur zusätzlichen Angabe einer Dosierung, die größer als eine Einheit (1/1 Tablette) ist, dagegen führt ein Druck auf den Pfeil 44 beispielsweise zu ½ Tablette, ein erneuter Druck auf Pfeil 44 zu % Tablette und dann bei einem erneuten Druck auf Pfeil 44 zu 1/8 Tablette. Im entgegen gesetzten Sinne führt ein Druck auf den gegenüberliegenden Pfeil 42 wieder zu ¼ Tablette, dann zu ½ Tablette und weiter über 1/1 Tablette zu 1 ½ Tabletten. Mit Taste 46 ist diese Einstellung registrierbar, z. B. "Medikament I: ½ Tablette eingenommen".

Zur beschleunigten Auswahl eines Medikamentes bzw. einer Befindlichkeit ist auch eine andere Vorgehensweise möglich: ein Druck auf den Pfeil 45 führt zu "Medikamentenwahl", Druck auf den Pfeil 42 ermöglicht ein "Rollen" in der Liste der Medikamente nach oben, Druck auf den Pfeil 44 ein Rollen nach unten. Eine Bestätigung erfolgt durch Drücken der Taste 46; dadurch wird durch Drücken des Pfeils 42 bzw. 44 wieder eine Dosiswahl möglich, die mit Taste 46 gespeichert wird. Ein Druck auf den Pfeil 43 führt dagegen beispielsweise zu Befindlichkeiten, Druck auf die Tasten 42 oder 44 ermöglicht wieder eine Auswahl der speziellen Befindlichkeit aus der Liste, Druck auf die Taste 46 führt zur Fixierung dieser Befindlichkeit, die bezüglich ihrer Intensität mit Pfeil 42 oder 44 wieder bewertet werden kann. Eine Abspeicherung kann mit Taste 46, eine Rücknahme der Registrierung mit Taste 47 erfolgen. Wird die Taste 47 ein weiteres Mal gedrückt, ist wieder der Modus zur Wahl der Befindlichkeiten aus der Liste eingestellt, so dass man mit Pfeil 42 oder 44 "Rollen" kann. Eine Registrierung ist immer dann erfolgt, wenn die Bestätigung nacheinander insgesamt zweimal durchgeführt wurde.

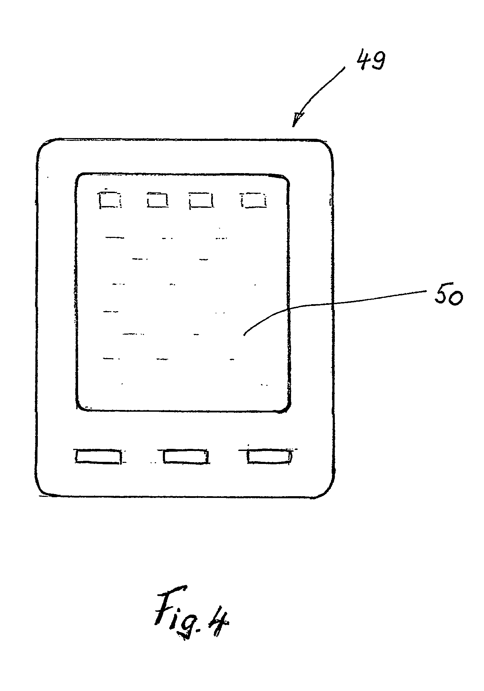

**Fig. 4** zeigt eine weitere Ausführungsform für ein Gerät 49 zur Eingabe von Befindlichkeitswerten - als Alternative zu dem zuvor beschriebenen Gerät 40 gemäß Fig. 3. Das Eingabegerät 49 entspricht einem tragbaren Rechner, einem so genannten hand-held-computer (oder auch PDA = personal digital assistant), handelsüblich unter der Bezeichnung "Palm"- Dieses Gerät 49 weist eine Berührfläche 50, einen so genannten touch screen auf, welcher Eingaben und Eintragungen sowie Aufzeichnungen durch Berühren mit einem speziellen Stift oder direkt mit den Fingern zulässt und speichert. Außerdem können durch "Anticken" bestimmte Felder oder Benutzeroberflächen mit für den Patienten relevanten Daten aufgerufen werden. Im Prinzip können über dieses Gerät dieselben Eingaben erfolgen wie über das bekannte Gerät des Anmelders.

Ebenso wie das o. e. Gerät 9 (vgl. Seite 6, 7) sind auch die Geräte 40 (vgl. Seite 9) und 49 in der Lage, die eingegebenen, registrierten und gespeicherten Befindlichkeitswerte direkt an die Zentrale 10 oder indirekt über die Zwischenstation 10a (vgl. Seite 7) zu funken.

## Patentansprüche

1. Vorrichtung zur Messung, Aufzeichnung und Speicherung von physiologischen, mittels Sensoren (2, 3, 4, 5, 6) am Körper eines Patienten (1) gemessenen Daten und von vom Patienten eingegebenen Befindlichkeitswerten, wobei die Vorrichtung eine Alarmvorrichtung (8) aufweist, die geeignet ist, bei Überschreiten von vorgegebenen Sollwerten für die physiologischen Daten einen ersten Alarm auszulösen wobei die Vorrichtung ein lesbares Display, eine hörbare Sprachaugabevorrichtung oder eine taktil wirksame Anweisungsvorrichtung beinhaltet, **dadurch gekennzeichnet dass** die Vorrichtung nach Auslösen des ersten Alarms den Patient durch die lesbare Anweisung auf dem Display, durch eine hörbare Sprachausgabe oder durch eine taktile Anweisung auffordert, seine akuten Befindlichkeitswerte einzugeben, und dass die Alarmvorrichtung (8) bei Überschreiten von vorgegebenen Sollwerten für die Befindlichkeitswerte einen zweiten Alarm auslöst.

2. Vorrichtung nach Anspruch 1, **dadurch gekennzeichnet, dass** der Patient aufgefordert wird, mindestens ein Medikament einzunehmen oder eine Therapie durchzuführen.

3. Vorrichtung nach Anspruch 1 oder 2, **dadurch gekennzeichnet dass** der Alarm durch ein akustisches oder visuelles oder taktiles oder ein anderes sensorielles Signal wirksam wird.

4. Vorrichtung nach einem der vorhergehenden Ansprüche, **dadurch gekennzeichnet dass** die Alarmvorrichtung (8) eine elektronische Vergleichseinheit aufweist, in welcher die von den Sensoren (2, 3, 4, 5, 6) gemessenen Werte und/oder Befindlichkeitsangaben empfangen und mit für den Patienten eingegebenen Sollwerten verglichen werden.

5. Vorrichtung nach einem der vorhergehenden Ansprüche, **dadurch gekennzeichnet, dass** die von den Sensoren (2, 3, 4, 5, 6) gemessenen Werte und/oder Befindlichkeitsangaben per Funk (drahtlos) an eine Empfängerstation der Alarmvorrichtung (8) übermittelt werden.

6. Vorrichtung nach einem der Ansprüche 1 bis 5, **dadurch gekennzeichnet, dass** die von den Sensoren (2, 3, 4, 5, 6) gemessenen Werte und die vom Patienten eingegebenen Befindlichkeitswerte an eine Zentrale (10) drahtlos übermittelt werden.

7. Vorrichtung nach Anspruch 6, **dadurch gekennzeichnet, dass** die gemessenen Werte und die eingegebenen Befindlichkeitswerte über eine Zwischenstation (10a) an die Zentrale (10) übermittelt werden.

8. Vorrichtung nach Anspruch 7, **dadurch gekennzeichnet, dass** die gemessenen Werte und die eingegebenen Befindlichkeitswerte von der Zwischenstation (10a) per Datenleitung an die Zentrale (10) übermittelt werden.

9. Vorrichtung nach einem der Ansprüche 6 bis 8, **dadurch gekennzeichnet, dass** die vorgegebenen Sollwerte für die physiologischen Daten und/oder die Befindlichkeitswerte von der Zentrale (10) durch drahtlose Übermittlung von Signalen änderbar sind.

10. Vorrichtung nach Anspruch 9, **dadurch gekennzeichnet, dass** die Übermittlung der Signale von der Zentrale (10) an die Zwischenstation (10a) drahtlos oder per Datenleitung erfolgt.

11. Vorrichtung nach einem der vorhergehenden Ansprüche, **dadurch gekennzeichnet, dass** die vom Patienten einzugebenden Befindlichkeitswerte über eine Multifunktionstaste (41) eines Registriergerätes (40) eingebbar sind.

12. Vorrichtung nach Anspruch 11, **dadurch gekennzeichnet, dass** die Eingaben des Patienten über zusätzliche Tasten (46, 47) registrierbar oder löschbar sind.

13. Vorrichtung nach einem der Ansprüche 1 bis 9, **dadurch gekennzeichnet, dass** ein Berührfeld (50), über welches die Befindlichkeitswerte und weitere für den Patienten relevante Daten durch Berühren, insbesondere durch einen Stift eingebbar sind.

14. Verfahren zur Überwachung des Gesundheitszustandes eines Patienten unter Verwendung einer Vorrichtung gemäß einem der vorhergehenden Ansprüche, wobei
- die mittels der Sensoren (2,3,4,5) gemessenen physiologischen Werte an die Alarmvorrichtung (8) übermittelt werden,
- bei Überschreiten vorgegebener Sollwerte ein Alarm ausgelöst wird und
- der Patient nach. Auslösung des Alarms zur Eingabe seiner akuten Befindlichkeitswerte aufgefordert wird.

15. Verfahren nach Anspruch 14, wobei die gemessenen und eingegebenen Werte zusätzlich an eine Zentrale (10) übermittelt werden.

16. Verfahren nach Anspruch 15, wobei die gemessenen und eingegebenen Werte über eine Zwischenstation (10a) an die Zentrale (10) übermittelt werden.

17. Verfahren nach Anspruch 14, 15 oder 16, wobei die Alarmsignale direkt oder indirekt an die Zentrale (10) übermittelt werden.

## Claims

1. Apparatus for measuring, recording and storing physiological data, measured on the body of a patient (1) by means of sensors (2, 3, 4, 5, 6), and the state of health entered by the patient, the apparatus having an alarm apparatus (8) which is suitable for triggering a first alarm if predetermined reference values for the physiological data are exceeded, the apparatus comprising a readable display, an audible speech output apparatus or an instruction apparatus effective by touch, **characterized in that** the apparatus uses the readable instruction on the screen, an audible speech output or a tactile instruction to request that the patient enter his or her acute state of health after the first alarm is triggered and **in that** the alarm apparatus (8) triggers a second alarm if predetermined reference values for the state of health are exceeded.

2. Apparatus according to Claim 1, **characterized in that** the patient is requested to take at least one medicament or carry out therapy.

3. Apparatus according to Claim 1 or 2, **characterized in that** the alarm becomes effective by an acoustic or visual or tactile or another sensorial signal.

4. Apparatus according to one of the preceding claims, **characterized in that** the alarm apparatus (8) has an electronic comparator in which the values measured by the sensors (2, 3, 4, 5, 6) and/or statements regarding the state of health are received and compared to reference values entered for the patient.

5. Apparatus according to one of the preceding claims, **characterized in that** the values measured by the sensors (2, 3, 4, 5, 6) and/or statements regarding the state of health are transmitted to a receiver station of the alarm apparatus (8) by radio waves (wirelessly).

6. Apparatus according to one of Claims 1 to 5, **characterized in that** the values measured by the sensors (2, 3, 4, 5, 6) and the state of health entered by the patient are transmitted wirelessly to a command centre (10).

7. Apparatus according to Claim 6, **characterized in that** the measured values and the entered state of health are transmitted to the command centre (10) via an intermediate station (10a).

8. Apparatus according to Claim 7, **characterized in that** a data line is used to transmit the measured values and the entered state of health to the command centre (10) from the intermediate station (10a).

9. Apparatus according to one of Claims 6 to 8, **characterized in that** the predetermined reference values for the physiological data and/or the state of health can be amended by the command centre (10) by wireless transmission of signals.

10. Apparatus according to Claim 9, **characterized in that** the transmission of the signals from the command centre (10) to the intermediate station (10a) is effected wirelessly or by a data line.

11. Apparatus according to one of the preceding claims, **characterized in that** the state of health to be entered by the patient can be input using a multifunction key (41) of a register unit (40).

12. Apparatus according to Claim 11, **characterized in that** the inputs of the patient can be registered or deleted using additional keys (46, 47).

13. Apparatus according to one of Claims 1 to 9, **characterized by** a touch field (50) by means of which the state of health and further data relevant to the patient can be entered by touch, in particular by using a pen.

14. Method for monitoring the physical condition of a patient using an apparatus according to one of the preceding claims, in which
- the physiological values measured by the sensors (2, 3, 4, 5) are transmitted to the alarm apparatus (8),
- an alarm is triggered if predetermined reference values are exceeded, and
- the patient is requested to input his or her acute state of health after the alarm is triggered.

15. Method according to Claim 14, in which the measured and entered values are additionally transmitted to a command centre (10).

16. Method according to Claim 15, in which the measured and entered values are transmitted to the command centre (10) via an intermediate station (10a).

17. Method according to Claim 14, 15 or 16, in which the alarm signals are transmitted to the command centre (10) directly or indirectly.

## Revendications

1. Dispositif pour mesurer, enregistrer et mémoriser des données physiologiques mesurées au moyen de capteurs (2, 3, 4, 5, 6) sur le corps d'un patient (1) et des valeurs d'état de santé ressenti saisies par le patient, le dispositif présentant un dispositif d'alarme (8) qui est conçu pour déclencher une première alarme en cas de dépassement de valeurs de consigne prédéfinies pour les données physiologiques, le dispositif comprenant un afficheur lisible, un dispositif d'émission vocale audible ou un dispositif de notification à action tactile, **caractérisé en ce que** le dispositif, après le déclenchement de la première alarme, invite le patient par le biais de la notification lisible sur l'afficheur, par une émission vocale audible ou par une notification tactile à saisir ses valeurs d'état de santé ressenti aiguës et que le dispositif d'alarme (8) déclenche une deuxième alarme en cas de dépassement de valeurs de consigne prédéfinies pour les valeurs d'état de santé ressenti.

2. Dispositif selon la revendication 1, **caractérisé en ce que** le patient est invité à absorber au moins un médicament ou à effectuer une thérapie.

3. Dispositif selon la revendication 1 ou 2, **caractérisé en ce que** l'alarme est matérialisée par un signal sonore ou visuel ou tactile ou un autre signal sensoriel.

4. Dispositif selon l'une des revendications précédentes, **caractérisé en ce que** le dispositif d'alarme (8) présente une unité de comparaison électronique dans laquelle les valeurs mesurées par les capteurs (2, 3, 4, 5, 6) et/ou les valeurs d'état de santé ressenti sont réceptionnées et comparées avec des valeurs de consigne saisies pour le patient.

5. Dispositif selon l'une des revendications précédentes, **caractérisé en ce que** les valeurs mesurées par les capteurs (2, 3, 4, 5, 6) et/ou les valeurs d'état de santé ressenti sont transmises par voie hertzienne (sans fil) à une station réceptrice du dispositif d'alarme (8).

6. Dispositif selon l'une des revendications 1 à 5, **caractérisé en ce que** les valeurs mesurées par les capteurs (2, 3, 4, 5, 6) et les valeurs d'état de santé ressenti saisies par le patient sont transmises sans fil à une centrale (10).

7. Dispositif selon la revendication 6, **caractérisé en ce que** les valeurs mesurées et les valeurs d'état de santé ressenti saisies sont transmises à la centrale (10) par le biais d'une station intermédiaire (10a).

8. Dispositif selon la revendication 7, **caractérisé en ce que** les valeurs mesurées et les valeurs d'état de santé ressenti saisies sont transmises de la station intermédiaire (10a) à la centrale (10) par ligne de données.

9. Dispositif selon l'une des revendications 6 à 8, **caractérisé en ce que** les valeurs de consigne prédéfinies pour les données physiologiques et/ou les valeurs d'état de santé ressenti peuvent être modifiées par la centrale (10) par une transmission sans fil de signaux.

10. Dispositif selon la revendication 9, **caractérisé en ce que** la transmission des signaux de la centrale (10) à la station intermédiaire (10a) est réalisée sans fil ou par ligne de données.

11. Dispositif selon l'une des revendications précédentes, **caractérisé en ce que** les valeurs d'état de santé ressenti à saisir par le patient peuvent être saisies par le biais d'une touche multifonction (41) d'un appareil d'enregistrement (40).

12. Dispositif selon la revendication 11, **caractérisé en ce que** les saisies du patient peuvent être enregistrées ou effacées par le biais de touches supplémentaires (46, 47).

13. Dispositif selon l'une des revendications 1 à 9, **caractérisé par** une zone tactile (50) par le biais de laquelle les valeurs d'état de santé ressenti ainsi que d'autres données pertinentes concernant le patient peuvent être saisies par un contact, notamment par un stylet.

14. Procédé de surveillance de l'état de santé d'un patient en utilisant un dispositif selon l'une des revendications précédentes, les valeurs physiologiques mesurées au moyen des capteurs (2, 3, 4, 5) étant transmises au dispositif d'alarme (8), une alarme étant déclenchée en cas de dépassement de valeurs de consigne prédéfinies et le patient étant invité, après le déclenchement de l'alarme, à saisir sa valeur d'état de santé ressenti actuelle.

15. Procédé selon la revendication 14, les valeurs mesurées et saisies étant en plus transmises à une centrale (10).

16. Procédé selon la revendication 15, les valeurs mesurées et saisies étant transmises à une centrale (10) par le biais d'une station intermédiaire (10a).

17. Procédé selon la revendication 14, 15 ou 16, les signaux d'alarme étant transmis directement ou indirectement à la centrale (10).
